# EUROPEAN PATENT APPLICATION

(11) **EP 3 949 921 A2**
(43) Date of publication of application: **09.02.2022**
(21) Application number: 21188446.5
(22) Date of filing: 29.07.2021
(51) Int. Cl.: A61F 13/44, A61F 13/84, A61B 5/00

(54) **INTELLIGENT MEDICAL GAUZE PAD**

(30) Priority: 07.08.2020 TW 109126918
(71) Applicant: AI Biomaterial Healthtech Ltd., Hong Kong 999077 (HK)
(72) Inventor: HO, Yen-Yi, 32450 Taoyuan City (TW)
(74) Representative: Viering, Jentschura & Partner mbB Patent- und Rechtsanwälte

(57) **Abstract**

An intelligent medical gauze pad is disclosed. The intelligent medical gauze pad is used with a sensing device. The intelligent medical gauze pad includes a main body and an identification module. The identification module is disposed on the main body. The identification module is used to sense a sensing signal emitted by the sensing device and return an identification signal such that the sensing device can locate the intelligent medical gauze pad according to the identification signal.

## Description

### BACKGROUND OF THE INVENTION

### 1. Field of the Invention

The present invention relates to an intelligent medical gauze pad, and more particularly, to an intelligent medical gauze pad which can return an identification signal.

### 2. Description of the Related Art

During a surgical operation, a medical gauze pad may be temporarily placed in the patient's body and then removed before the operation is completed and the wound is sutured. If the medical gauze pad is not removed, it may cause pain, infection, abscess, fistula formation, intestinal obstruction and other problems. In the prior art, it is necessary to rely on the medical staff to carefully check the number of medical gauze pads before and after the operation to avoid loss of a medical gauze pad. If a medical gauze pad is lost, it is necessary to locate the missing medical gauze pad in the patient's body by any means necessary, even radiological examination. Consequently, the loss of a medical gauze pad will not only cause harm to the patient but also consume valuable medical resources.

Therefore, it is necessary to invent a novel intelligent medical gauze pad to solve the deficiencies of the prior art techniques.

### SUMMARY OF THE INVENTION

It is a main object of the present invention to provide an intelligent medical gauze pad which can return an identification signal.

In order to achieve the above object, the present invention discloses an intelligent medical gauze pad used with a sensing device. The intelligent medical gauze pad includes a main body and an identification module. The identification module is disposed in the main body. The identification module is used to sense a sensing signal emitted by the sensing device and return an identification signal such that the sensing device can locate the intelligent medical gauze pad according to the identification signal.

According to an embodiment of the present invention, the identification module comprises a memory module for storing usage data, wherein the identification signal corresponds to the usage data such that the sensing device can read the identification signal and identify the intelligent medical gauze pad.

According to an embodiment of the present invention, the identification module comprises a wireless signal transmission module signally connected with a blockchain network, by means of which the usage data is recorded in the blockchain network for traceability.

According to an embodiment of the present invention, the usage data record may include a type, a sequence number, a manufacturing date, a manufacturing location, a serial number, a batch number or an expiry date of the intelligent medical gauze pad.

According to an embodiment of the present invention, the identification module can be an radio frequency identification (RFID) tag, a near field communication (NFC) tag, or a flexible chip.

According to an embodiment of the present invention, the main body is a chitin dressing or other dressing with an antibacterial function.

According to an embodiment of the present invention, the main body is a hemostatic cotton pad or a hemostatic gauze pad.

According to an embodiment of the present invention, the main body is a piece of artificial skin.

### BRIEF DESCRIPTION OF THE DRAWINGS

Fig. 1 illustrates a schematic diagram of the appearance of an intelligent medical gauze pad of the present invention;
Fig. 2 illustrates an operational diagram of the intelligent medical gauze pad of the present invention in connection with an external device.

### DETAILED DESCRIPTION OF THE PREFERRED EMBODIMENTS

In order to make the structure and characteristics as well as the effectiveness of the present invention to be further understood and recognized, the detailed description of the present invention is provided as follows along with embodiments and accompanying figures.

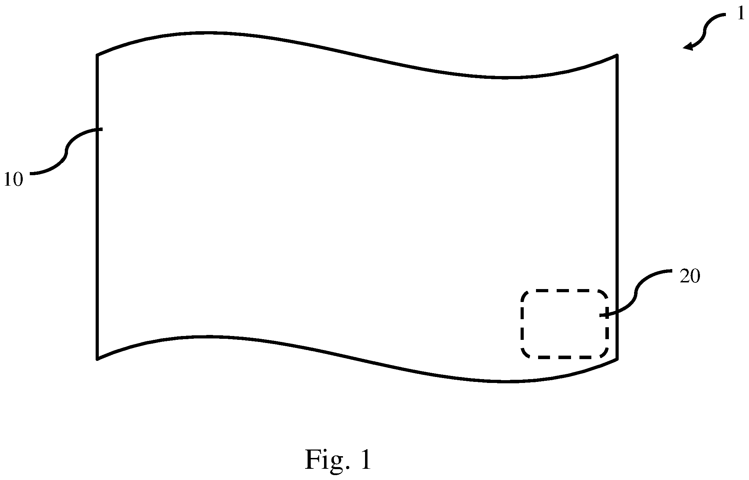

In the following, please refer to Fig. 1 for a schematic diagram of the appearance of an intelligent medical gauze pad of the present invention.

In an embodiment of the present invention, the intelligent medical gauze pad 1 includes a main body 10 and an identification module 20. The main body 10 can be a chitin dressing, hemostatic cotton pad, piece of artificial skin, or hemostatic gauze pad used in surgical procedures, and the main body is not limited to the materials described above. The present invention does not limit the material used for the main body 10, which means that other materials with antibacterial effects can be used. The identification module 20 can be a radio frequency identification (RFID) tag or a near field communication (NFC) tag disposed on the main body 10, such as attached to one side of the main body 10 with an adhesive or sewn into the inside of the main body 10.

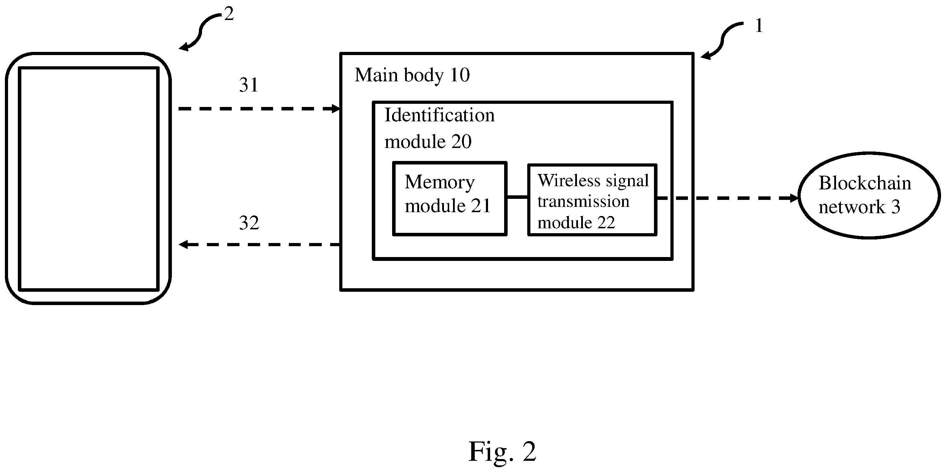

Next, please refer to Fig. 2 for an operational diagram of the intelligent medical gauze pad of the present invention in connection with an external device.

In another embodiment of the present invention, the identification module 20 can comprise a memory module 21 for storing usage data such as a type, a sequence number, a manufacturing date, a manufacturing location, a serial number, a batch number or an expiry date of the intelligent medical gauze pad 1. Since the identification module 20 can be an RFID tag, an NFC tag or a flexible chip, different tags of the identification modules 20 can also have different identification codes; therefore, these different identification codes can be used as the serial number of the intelligent medical gauze pad 1, but the present invention is not limited thereto. In an embodiment of the present invention, the intelligent medical gauze pad 1 is set to work with a sensing device 2, which can be a dedicated device, a smart phone, a wearable device, etc. The identification module 20 is used to sense a sensing signal 31 emitted by the sensing device 2 in a non-contact manner so as to return an identification signal 32 to the sensing device 2 according to the sensing signal 31. In this way, the sensing device 2 can locate the position of the identification module 20 that emits the sensing signal 31 such that the intelligent medical gauze pad 1 can be easily located. When a plurality of intelligent medical gauze pads 1 is placed at the same time, the sensing device 2 can use the plurality of identification signals 32 to count the number of the plurality of intelligent medical gauze pads 1 to avoid omissions.

In another embodiment of the present invention, the identification signal 32 can also correspond to the usage data; that is, the identification signal 32 returned by the identification module 20 can include all or part of the usage data. Therefore, after the sensing device 2 reads the identification signal 32, a medical worker can use the identification signal 32 to identify the type or number of the one or plurality of intelligent medical gauze pads 1 for further management. The intelligent medical gauze pad 1 can reduce the workload of medical staff and lower the chance of omission, and it can also prevent the problem of postoperative infection caused by a medical gauze pad remaining in a patient's body after surgery.

Furthermore, in another embodiment of the present invention, the identification module 20 may also include a wireless signal transmission module 22. The wireless signal transmission module 22 is used to signally connect with a blockchain network 3 via a wireless communication protocol, by means of which the usage data of the intelligent medical gauze pad 1 can be automatically or passively recorded in the blockchain network 3 for traceability. That is, the wireless signal transmission module 22 can record the type, serial number, manufacturing date, or manufacturing location mentioned above in the blockchain network 3. In this way, the source and history of the intelligent medical gauze pad 1 can be clearly recorded and managed by the blockchain network because the blockchain network is traceable and is not easily tampered with, making it easier to manage the records during surgery. For example, during a surgery, it is easy to record the time when the intelligent medical gauze pad 1 is placed and when it is removed, the order of use of a plurality of medical gauze pads 1, and the time span of use, and it is also easy to clearly record the usage data in the electronic medical record on the blockchain network 3, making the whole operating process more complete and smooth; therefore, the present invention can better protect the safety of patients and the integrity of the surgery.

From the above description, it can be seen that the intelligent medical gauze pad 1 can provide a more convenient management function and clearly record the usage history through the identification module 20 disposed therein, which can also help reduce errors made by medical personnel.

It is noted that the above-mentioned embodiments are only for illustration. It is intended that the present invention cover modifications and variations of this invention provided they fall within the scope of the following claims and their equivalents. Therefore, it will be apparent to those skilled in the art that various modifications and variations can be made to the structure of the present invention without departing from the scope or spirit of the invention.

## Claims

1. An intelligent medical gauze pad (1) used with a sensing device (2), wherein the intelligent medical gauze pad (1) comprises:
a main body (10) comprising a hemostatic cotton or a hemostatic gauze, and
an identification module (20) disposed in the main body (10), wherein the identification module (20) is used to sense a sensing signal (31) emitted by the sensing device (2) and return an identification signal (32) such that the sensing device (2) can locate the intelligent medical gauze pad (1) according to the identification signal (32), and the identification module (20) comprises a wireless signal transmission module (22) signally connected with a blockchain network (3), by means of which usage data corresponding to the identification signal (32) can be recorded in the blockchain network (3) for traceability.

2. The intelligent medical gauze pad (1) as claimed in Claim 1, wherein the identification module (20) comprises a memory module (21) for storing the usage data such that the sensing device (2) can read the identification signal (32) and identify the intelligent medical gauze pad (1).

3. The intelligent medical gauze pad (1) as claimed in Claim 2, wherein the usage data record a type, a sequence number, a manufacturing date, a manufacturing location, a serial number, a batch number or an expiry date of the intelligent medical gauze pad (1).

4. The intelligent medical gauze pad (1) as claimed in Claim 1, wherein the identification module (20) can be an radio frequency identification (RFID) tag, a near field communication (NFC) tag, or a flexible chip.
